# EUROPEAN PATENT APPLICATION

(11) **EP 1 202 211 A2**
(43) Date of publication of application: **02.05.2002**
(21) Application number: 01309062.6
(22) Date of filing: 25.10.2001
(51) Int. Cl.: G06F 19/00

(54) **Genomic DNA analysis computer program**

(30) Priority: 26.10.2000 JP 2000327632; 23.03.2001 JP 2001084370
(71) Applicant: RIKEN, Wako-shi, Saitama 351-0198 (JP)
(72) Inventor: Yoshida, Shigeo, c/o Riken, Wako-shi, Saitama 351-0198 (JP); Matsuyama, Tomoki, c/o Riken, Wako-shi, Saitama 351-0198 (JP); Abe, Tomoko, c/o Riken, Wako-shi, Saitama 351-0198 (JP); Ebisuzaki, Toshikazu, c/o Riken, Wako-shi, Saitama 351-0198 (JP)
(74) Representative: Brasnett, Adrian Hugh

(57) **Abstract**

The present invention makes possible the easy production of a control two-dimensional electrophoresis pattern, a target for comparison, under various conditions, and perform quick and accurate analysis.

The present invention relates to a program which allows a computer to execute the processes of: producing a control two-dimensional electrophoresis pattern based on genomic nucleotide sequence information, comparing the control two-dimensional electrophoresis pattern and a target two-dimensional electrophoresis pattern obtained by performing two-dimensional electrophoresis using target genomic DNA, and detecting a difference of spot positions between the control two-dimensional electrophoresis pattern and the target two-dimensional electrophoresis pattern.

## Description

### FIELD OF THE INVENTION

The present invention relates to a computer program which analyzes a two-dimensional electrophoresis pattern using genomic DNA; and a method for analyzing genomic DNA using a two-dimensional electrophoresis pattern.

### BACKGROUND OF THE INVENTION

In recent years, in order to carry out more rapid genome analysis of higher organisms, the development of genome scanning techniques has been progressing rapidly. Genome scanning is a scan to analyze loci (gene loci) or copy number thereof at a high speed and thereby detect the physical condition of genomic DNA throughout a genome as a whole. Genome scanning enables high speed scanning of signals relating to, and thereby determination of, a gene position on a genome gene map or a chromosomal compartment, which a DNA region encoding one gene product occupies. In this genome scanning, it is extremely important what kind of mark is raised on a genome, what kind of signal is used to detect any mark on a genome, and how to detect and analyze as many marks as possible. This mark is called a landmark, and analysis of information regarding position of the landmark on a chromosome could be an important basic technique to produce a linkage map or a physical map of genome. Therefore, application of the genome scanning to two or more genomic DNA molecules to make a comparison enables detection of changes in genomic DNA molecules such as deletion, amplification and translocation.

RLGS (Restriction Landmark Genomic Scanning) is a method which comprises labeling, as a landmark, a portion of genomic DNA cleaved with restriction enzymes, developing the labeled genomic DNA fragment by two-dimensional electrophorcsis, and detecting it using the obtained pattern. It can be said that this RLGS method is a technique by which change in genomic DNA can be detected at a high speed in that a large number of landmarks can be detected at one time by this method.

In this RLGS, a control two-dimensional electrophoresis pattern is required for comparison with the two-dimensional electrophoresis pattern obtained from target genomic DNA. A control two-dimensional electrophoresis pattern can be obtained by similarly performing two-dimensional electrophoresis using genomic DNA extracted from a wild type cell. In RLGS method, a process of obtaining a control two-dimensional electrophoresis pattern is complicated, and comparison using the obtained control two-dimensional electrophoresis pattern is also complicated, Furthermore, there is a problem that enormous effort and complex techniques are required to clone a spot.

### SUMMARY OF THE INVENTION

Thus, the present invention is directed to provide a computer program, which enables a control two-dimensional electrophoresis pattern and a target for comparison to be produced easily under various conditions, and the two-dimensional electrophoresis pattern to be analyzed using genomic DNA. Moreover, the present invention is directed to providing a method for analyzing genomic DNA, in which rapid and accurate analysis can easily be carried out by producing a control two-dimensional clectrophoresis pattern under various conditions.

The present invention, whereby the above objects may be accomplished, may include one or more of the following features in preferred embodiments.
(1) A program which allows a computer to execute the processes of:
   producing a control two-dimensional electrophoresis pattern based on genomic nucleotide sequence information,
   comparing the control two-dimensional electrophoresis pattern and a target two-dimensional electrophoresis pattern obtained by performing two-dimensional electrophoresis using target genomic DNA, and
   detecting a difference in spot position between the control two-dimensional electrophoresis pattern and the target two-dimensional electrophoresis pattern.
(2) The program according to (1) above, wherein, in the process of producing the control two-dimensional electrophoresis pattern, the control two-dimensional electrophoresis pattern is produced by detecting the recognition sequence of a first restriction enzyme and the recognition sequence of a second restriction enzyme in the genomic nucleotide sequence information, and basing on a nucleotide sequence flanked by the first restriction enzyme recognition sequence and the second restriction enzyme recognition sequence and another nucleotide sequence flanked by the two first restriction enzyme recognition sequences.
(3) The program according to (2) above, wherein the first restriction enzyme and the second restriction enzyme are selected from methylation insensitive restriction enzymes.
(4) The program according to (2) above, wherein the first restriction enzyme and the second restriction enzyme are selected from methylation sensitive restriction enzymes.
(5) The program according to (1) above, which comprises a process of obtaining the genomic nucleotide sequence information by means of a communication line network.
(6) The program according to (1) above, wherein, in the process of producing the control two-dimensional electrophoresis pattern, a plurality of spots are produced based on genomic nucleotide sequence information and these spots are linked to gene loci information on a genome.
(7) The program according to (1) above, wherein, in the process of producing a control two-dimensional electrophoresis pattern based on a genomic nucleotide sequence information, abnormal information comprised in the genomic nucleotide sequence information is detected.
(8) The program according to (7) above, which links the detected abnormal information to a spot comprised in the produced two-dimensional electrophoresis pattern in order to memorize the information.
(9) A method for analyzing genomic DNA which comprises the steps of:
   producing a two-dimensional electrophoresis pattern by performing two-dimensional electrophoresis using a target genomic DNA,
   comparing the two-dimensional electrophoresis pattern and a control two-dimensional electrophoresis pattern produced based on genomic nucleotide sequence information, and
   detecting a difference in spot position between the control two-dimensional electrophoresis pattern and the target two-dimensional electrophoresis pattern.
(10) The method for analyzing genomic DNA according to (9) above, which comprises a step of extracting the target genomic DNA from plant cells.
(11) The method for analyzing genomic DNA according to (9) above, wherein the target genomic DNA is derived from a higher organism.
(12) The method for analyzing genomic DNA according to (9) above, wherein, in said step of producing the two-dimensional electrophoresis pattern of the target genomic DNA by the following steps (a) to (e):
   (a) treating genomic DNA with a first restriction enzyme,
   (b) adding a label to a site cleaved with the restriction enzyme,
   (c) performing a first-dimension fractionation on the obtained DNA fragment by electrophoresis,
   (d) after treating the DNA fragment fractioned in step (c) with a second restriction enzyme, performing a second-dimension fractionation, and
   (e) detecting a spot of the labeled DNA fragment fractioned in step (d).
(13) The method for analyzing genomic DNA according to (12) above, wherein, after the step (b), the obtained DNA fragment is treated with a restriction enzyme different from the first and second restriction enzymes and before the step (c) is performed.
(14) The method for analyzing genomic DNA according to (12) above, which comprises detecting the recognition sequences of the first and second restriction enzymes in genomic nucleotide sequence information, and producing the control two electrophoresis pattern based on these cleavage sites.
(15) The method for analyzing genomic DNA according to (12) above, wherein the step (b) is carried out by connecting one end of an adapter to the restriction enzyme cleavage site as well as adding the label to the other end of the adapter.
(16) The method for analyzing genomic DNA according to (9) above, wherein the control two-dimensional electrophoresis pattern has a plurality of spots produced based on genomic nucleotide sequence information and links these spots to gene loci information on a genome.
(17) The method for analyzing genomic DNA according to (9) above, which comprises detecting abnormal information comprised in genomic nucleotide sequence information before producing the control two-dimensional electrophoresis pattern based on the genomic nucleotide sequence information.
(18) The method for analyzing genomic DNA according to (17) above, which comprises linking the detected abnormal information to a spot comprised in the produced two-dimensional electrophoresis pattern.
(19) A method for identifying genes associated with mutant genes and/or mutant traits, which comprises detecting mutated sites in target genomic DNA by the method for analyzing genomic DNA according to any one of (9) to (18) above.
(20) A method for isolating genes associated with mutant genes and/or mutant traits, which comprises isolating a DNA fragment containing mutated sites detected by the identification method according to (19) above, from the two-dimensional electrophoresis pattern.
(21) A two-dimensional electrophoresis pattern, which has a plurality of spots produced based on genomic nucleotide sequence information and estimated from the genomic nucleotide sequence information.
(22) The two-dimensional electrophoresis pattern according to (21) above, wherein the plurality of spots are linked to gene loci information on a genome.
(23) The two-dimensional electrophoresis pattern according to (21) above, wherein the genomic nucleotide sequence information is nucoleotide sequence information obtained from a plant nuclear genome.
(24) The two-dimensional electrophoresis pattern according to (21) above, wherein abnormal information detected from the genomic nucleotide sequence information is linked to the plurality of spots.
(25) The two-dimensional electrophoresis pattern according to (21) above, wherein the genomic nucleotide sequence information is obtained from the genome of a higher organism.

Further aspects of the invention respectively provide a computer or linked computers operatively configured to implement any of the methods or programs described above and computer programming product or products (such as ROM, RAM, floppy discs, hard drives, optical compact discs, magnetic tapes, and other computer-readable media) carrying computer code for implementing any of the methods or programs described above.

### BRIEF DESCRIPTION OF THE DRAWINGS

Figure 1 shows a process of producing a two-dimensional electrophoresis pattern using a target genomic DNA.
Figure 2 shows a process of labeling using an adapter.
Figure 3 is a block diagram roughly showing a system executing a recording medium having the program of the present invention recorded thereon.
Figure 4 is a flow chart showing a process of executing the program.
Figure 5 shows a control two-dimensional electrophoresis pattern produced based on the nucleotide sequence information of *Arabidopsis thaliana* chloroplast DNA.
Figure 6 shows a target two-dimensional electrophoresis pattern produced using *Arabidopsis thaliana* chloroplast DNA.
Figure 7 shows a control two-dimensional electrophoresis pattern produced based on the nucleotide sequence information of *Arabidopsis thaliana* chomrosomal DNA.
Figure 8 is a diagrammatic sketch showing an example of an actual analysis with a control two-dimensional electrophoresis pattern.
Figure 9 is a diagrammatic sketch showing an example of the abnormal information obtained by detection of the abnormal information in nucleotide sequence information regarding *Arabidopsis thaliana* nuclear genome.

### Sequence Listing Free Text

In SEQ ID NO: 1, n denotes a, g, c or t (location: the 4th nucleotide to the 9^{th} nucleotide).

### Description of Symbols

501: CPU
503: RAM
504: Input portion
505: Sending/Receiving portion
506: Output portion
507: HDD
508: CD-ROM drive
509: CD-ROM
510: Database

### DETAILED DESCRIPTION OF THE INVENTION

The present invention is described in detail below.

The program of the present invention may be recorded in an information recording medium such as a flexible disk, an optical disk, a magneto-optical disk, a phase change optical disk and a hard disk, or may be transmitted from a server by means of a communication line network such as the internet. The present program recorded in an information recording medium or transmitted from a server is able to allow a computer to execute the following processes.

### This program comprises the following processes:

A process of producing a control two-dimensional electrophoresis pattern based on genomic nucleotide sequence information (see process 1 set forth below).

A process of comparing the above control two-dimensional electrophoresis pattern and a target two-dimensional electrophoresis pattern obtained by performing two-dimensional electrophoresis using target genomic DNA (see process 2 set forth below).

That is to say, this program allows a computer to compare the two-dimensional electrophoresis pattern obtained from genomic DNA extracted from an analysis target with a control two-dimensional electrophoresis pattern. In process 1 of this program, first, a control two-dimensional electrophoresis pattern is obtained. In process 2 of this program, using a separately obtained two-dimensional electrophoresis pattern of the analysis target, the target two-dimensional electrophoresis pattern is compared with the control two-dimensional electrophoresis pattern.

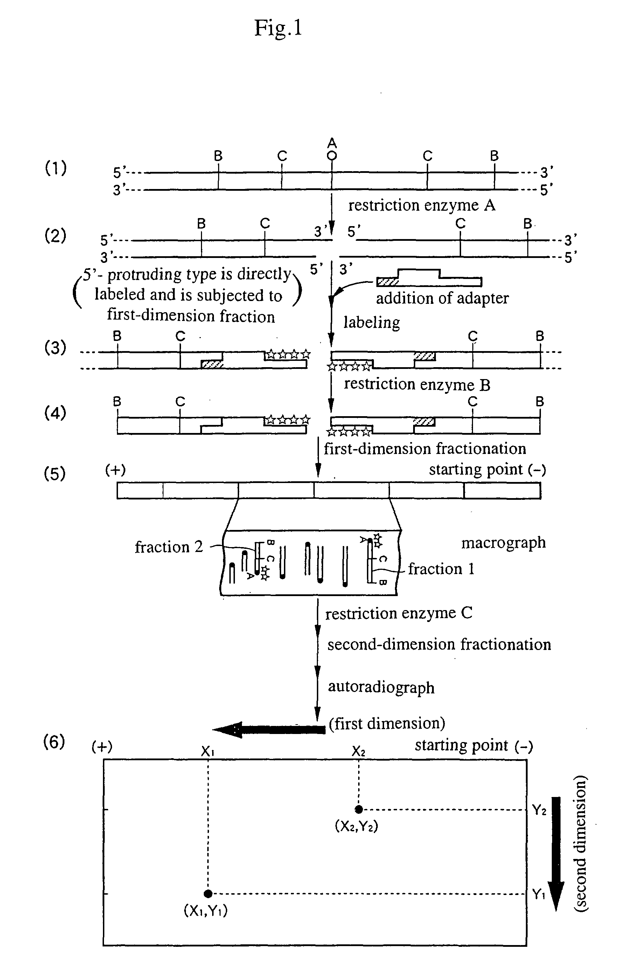

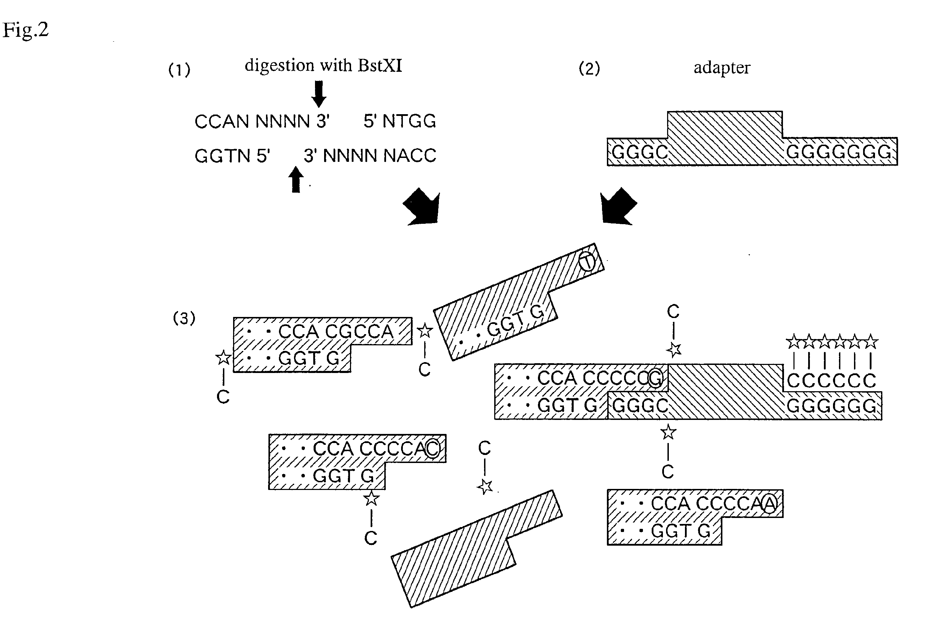

First, a method of obtaining the two-dimensional electrophoresis pattern of a target genomic DNA is described. For the two-dimensional electrophoresis pattern, a restriction enzyme recognition site is used as a mark (a landmark) and a method involving detection of the landmark as a signal (Restriction Landmark Genomic Scanning Method (RLGS method); Hatada, I. *et al., Proc. Natl. Acad. Sci.,* USA, 88, 9523-9527, 1991) is applied as a basic concept. With regard to analysis of plant genomic DNA, Matsuyama *et al*., *Plant. Mol. Biol. Rep.* 18:331-338, 2000 is applied as a basic idea. The two-dimensional electrophoresis pattern of a target genomic DNA can be obtained according to the following method.
(1) Treatment for genomic DNA with a first restriction enzyme
   The target genomic DNA of the analysis method of the present invention is not particularly limited, and genomic DNA molecules derived from either a plant or other types of organisms (animals, bacteria and yeast etc.) can be used. Examples of genomic DNA molecules include ones derived from higher organisms. Examples of genomic DNA molecules derived from plants include ones derived from rice, tobacco, *Arabidopsis,* wheat and tomato etc., and ones from rice or *Arabidopsis* are preferable.
   Examples of genomic DNA molecules derived from animals include ones derived from human, mouse and nematode etc. Examples of genomic DNA molecules derived from bacteria include ones derived from *Bacillus subtilis* and *Escherichia coli.*
   In the program and the DNA analysis method of the present invention, it is particularly preferable to use the above stated plant-derived genomic DNA as a target to be analyzed. The plant-derived genomic DNA can be obtained by performing protein removal and polysaccharide removal etc. on a plant according to a conventionally known method (Dellaporta *et al., Plant Mol. Biol. Rep. 1: 19-21,* 1983).
   In this "treatment of genomic DNA with a first restriction enzyme", first, extracted genomic DNA is cleaved with a first restriction enzyme (which is an enzyme cleaving site "A" in Figure 1(1), and is referred to as restriction enzyme A). Examples of restriction enzyme A include what are known as rare cutter restriction enzymes recognizing 6 to 8 nucleotides, which cleave to produce fragments having an average length of over 100kb, i.e. restriction enzymes which recognize restriction enzyme sites existing only at intervals that are on average more than 100kb.
   As the above-stated restriction enzyme A, there is used either a restriction enzyme that cleaves DNA such that the 5'-end of a restriction enzyme site generated by cleavage can be the protruding cohesive end (which is referred to as 5'-protruding type) or a restriction enzyme that cleaves DNA such that the 3'-end of a restriction enzyme site generated by cleavage can be the protruding cohesive end (which is referred to as 3'-protruding type). Furthermore, the length of a protruding portion is one nucleotide or more, and to obtain sufficient signal length, an enzyme cleaving DNA such that an the protruding portion can be 2 nucleotides or more is preferable. Examples of such restriction enzyme A include *Not*I, *Bss*HII and *Acc*III etc. for 5'-protruding type, and *Bst*XI, *Bgl*I and *Mwo*I etc. for 3'-protruding type.
   Especially where plant-derived genomic DNA is used as a target, methylation insensitive restriction enzymes are preferably used as the first restriction enzymes. Due to the use of methylation insensitive restriction enzymes, genomic DNA which has methylated cytosine, such as genomic DNA derived from plant cells, can also be cleaved with certainty. Examples of methylation insensitive restriction enzyme include *Acc*III and *Pac*I etc.
(2) labeling to restriction enzyme cleavage sites
   A label is added to sites cleaved with the above-stated restriction enzyme A. The addition of a label is carried out by filling previously labeled nucleotides (a sequence for labeling) in sites cleaved with restriction enzyme A, using sequenase (T7 DNA polymerase). Accordingly, in this case, to become a reaction target of sequenase, it is preferable to adopt as the above-stated restriction enzyme A, a restriction enzyme which cleaves DNA so that the 5'-end of a cleavage site can be the protruding cohesive end (referred to as 5'-protruding type).
   Examples of labeling substances include radioactive isotopes such as [α -³²P]dCTP and [ α -³²P]dGTP, and fluorescent dyes such as tetramethyl-rhodamine-6-dUTP and fluorescein-12-dUTP etc., and the labeling substance can be selected from these substances arbitrarily.
   When adding a label, an adapter may be added to a site cleaved with restriction enzyme A, and then a label may be added to the adapter. Regarding the adapter, one side thereof is designed as a sequence capable of connecting to the cleaved site of the above-stated restriction enzyme A (referred to as a combining sequence), whereas the other side of the adapter is designed as a sequence for labeling the adapter (referred to as a labeling sequence). By making the 5'-end of the labeling sequence of an adapter, the protruding cohesive end, it can be a reaction target of sequenase, and thereby allow addition of a label to the labeling sequence.
   Thus, where a label is added by means of an adapter, a preferred restriction enzyme A is one cleaving DNA so that the 3'-end of a cleavage site can be a protruding cohesive end, that is, a 3'-protruding type is preferable. In this case, fill-in by sequenase (T7 DNA polymerase) is prevented at the site cleaved with restriction enzyme A. In other words, the combining sequence of an adapter is preferably 5'-terminus protruding cohesive terminus.
   Herein, each of a combining sequence and a labeling sequence is constituted by a single strand. A sequence located between a combining sequence and a labeling sequence is constituted by a double strand consisting of 5 to 45 nucleotides. Taking facilitation of cloning into consideration, a double strand consisting of 20 to 35 nucleotides is preferable. A combining sequence can be designed, corresponding to the recognition sequence of the above restriction enzyme A. Further, a labeling sequence and a sequence in a double-stranded region can be designed arbitrarily.
   Since the length of a labeling sequence can be designed arbitrarily, the longer the length of a labeling sequence, the higher the detectability that can be obtained. However, if the length of a labeling sequence is too long, a secondary structure of DNA (e.g. a stem loop or hairpin structure) may be formed. Hence, a labeling sequence preferably consists of 2 to 10 nucleotides so that these secondary structures are not formed.
   As stated above, where a label is directly added to the cleavage site of restriction enzyme A, since it has a low spot detection ability to tobacco, *Arabidopsis* and the like, it is difficult to apply to all genomic DNA molecules. However, if a label is added by means of an adapter, it can be applied to all genomic DNA molecules, and detection ability thereof can be improved.
   Furthermore, where an adapter is used, *Bst*XI which has N (N represents A, G, C or T) in its recognition sequence may be used as restriction enzyme A. Where a 3'-protruding type such as *Bst*XI is used as restriction enzyme A, it does not become a reaction target of sequenase (T7 DNA polymerase) when labeling is carried out as described later, and so the use of 3'-protruding type can prevent fragments other than a target DNA fragment from being labeled. Apart from *Bst*XI, *Bgl*I and *Mwo*I, there exist a large number of 3'-protruding type of enzymes producing a protruding portion of two or more nucleotides (e.g. *Alw*NI, *Ban*II, *Bsi*EI, *Bsi*HKAI, *Bsl*I, *Bsm*I, *Bsp*1286I, *Bsr*DI, *Dra*III, *Drd*I, *Pfl*MI, *Sfi*I, *Tsp*RI, *Bpm*I, *Bse*RI, *Bsg*I, *Eco*57I), and any of these enzymes can be applied in the method of the present invention.
   For example, where *Bst*XI is used as restriction enzyme A, *Bst*XI recognizes the following sequences and cleaves DNA at a position between the 8^{th} N and the 9^{th} N (a position shown in ∗) so that 4 nucleotides protrude at the 3'-end (see underlines) (Figure 2(1)).
   Sense strand: 5'-CCANNNNN ∗ NTGG-3' (SEQ ID NO: 1)
   Antisense strand: 3'-GGTN ∗ NNNNNACC-5'

   Since N may be any of A, G, C and T, 4 sequences of a protruding portion of an adapter are designed, considering all types of combinations (4⁴=256 possible combinations). In this case, depending on the type of genomic DNA or the objects of analysis (specifically, detection of a mutated region in an *Arabidopsis* mutant, etc.), the 4 nucleotides of the a protruding portion can be specified so as to produce appropriate numbers of combinations. For example, Figure 2(2) shows "GGGC" as a combining sequence of an adapter. In this case, the adapter combines with one type of fragment having "CCCG" as a protruding sequence (i.e. a fragment having a sequence of which protruding portion is only "CCCG") among fragments obtained by cleavage of *Bst*XI (Figure 2(3)). Similarly, where a combining sequence of an adapter is "GGGS" (S shows G or C), the adapter combines with two types of fragments having a protruding sequence that is "CCCG" or "CCCC" from among fragments cleaved with *Bst*XI. Accrodingly, where *Bst*XI is used as restriction enzyme A, 1 to 256 possible combinations of landmark site(s) can arbitrarily be selected depending on the manner of selecting the combining sequence of an adapter.
   An adapter can be prepared using a common chemical synthesis device (e.g. DNA/RNA synthesizer, model 394, PE Applied Biosystems). Then, the adapter is treated with DNA ligase and connected to genomic DNA.
(3) Treatment for genomic DNA with a second restriction enzyme
   Where the length of a fragment obtained by cleavage of the restriction enzyme A is 100kb or more, the fragment cannot directly be subjected to fractionation by electrophoresis etc. Thus, the second restriction enzyme treatment is performed to obtain further shorter fragments from the fragments obtained by cleavage of the restriction enzyme A (Figure 1(4)). As a second restriction enzyme, one which recognizes 6 nucleotides can be used, such that when cleaving fragments having an average length of several to several tens of kbare produced, i.e. restriction enzymes which recognize restriction enzyme sites existing at intervals that are on average several to several tens of kb (this is an enzyme cleaving site B in Figure 1, and referred to as restriction enzyme B). Examples of restriction enzyme B include *Eco*RV and *Dra*I etc.
   Where the length of a fragment obtained by cleaving with restriction enzyme A is on average several to several tens of kb, the fragment may directly be subjected to fractionation by electrophoresis, and so the second restriction enzyme treatment is not needed.
(4) First-dimension fractionation
   After treatment with restriction enzyme B, each fragment is subjected to first-dimension fractionation (Figure 1(5)). First-dimension fractionation is carried out using a slim capillary tube having a diameter of 3 to 4.5mm and a length of about 60cm. The fragments treated in the above (3) are poured into the origin point of the tube and subjected to fractionation. First-dimension fractionation is preferably carried out by about 0.7% to 1.0% agarose gel electrophoresis in the presence of 5% sucrose at room temperature (more preferably 22°C to 26°C) for 20 to 48 hours.
   In this first-dimension fractionation step, fragments obtained by cleavage with restriction enzyme B are electrophoresed so that the length of each DNA fragment becomes shorter in order from the origin point (the starting point of first-dimension fractionation) to the terminus (the macrograph in Figure 1(5)). For example, in the macrograph of Figure 1(5), long fragment 1 is electrophoresed near the origin point, whereas short fragment 2 is electrophoresed pulling away from the origin point. Accordingly, the distance from the origin point reflects the length of a fragment.
(5) Treatment for genomic DNA with a third restriction enzyme
   After completion of the fractionation, the used tube is immersed in the third restriction enzyme solution to perform restriction enzyme treatment on the first-dimension fractionation product. The third restriction enzyme is one having higher cleavage frequency than restriction enzymes A and B, which when cleaving produce fragments having an average length of several hundreds of bp, i.e. restriction enzymes which recognize restriction enzyme sites existing at intervals that are on average about 300bp (this is an enzyme cleaving site C in Figure 1, and referred to as restriction enzyme C).
   As restriction enzyme C, one recognizing 4 to 6 nucleotides can be used, and examples of such restriction enzymes include *Mbo*I and *Hin*fl etc.
(6) Second-dimension fractionation
   When fragments are treated with restriction enzyme C, a fragment flanked by restriction enzyme recognition sites A and B (referred to as A-B fragment) is cleaved into one fragment flanked by restriction enzyme recognition sites A and C (referred to as A-C fragment) and another fragment flanked by restriction enzyme recognition sites C and B (referred to as B-C fragment), and the average chain length of each DNA fragment is several hundreds bp or less.
   These fragments are subjected to second-dimension fractionation. Examples of second-dimension method include a method involving 5% polyacrylamide gel electrophoresis (under conditions of 2v/cm for 20 to 24 hours) etc. Otherwise, a more accurate pattern can be obtained by performing an electrophoresis with 5% polyacrylamide gel containing 6M urea.
(7) Detection of spots
   Spots are detected by applying a technique suitable for the type of labeling substance. Examples of such techniques include autoradiography detection where ³²P is used as a labeling substance, and detection by fluorescent image analyzer (e.g. FMBIO II Multi-View, TaKaRa) where a fluorescent pigment is used. After two-dimensional fraction, B-C fragment is not labeled, and so no spots are produced.

If the positions of the thus obtained spots are expressed as distances from an origin point in the X and Y directions such as (X1, Y1), (X2, Y2), ... (Xn, Yn), then the X coordinate reflects the distance from the recognition site of restriction enzyme A to that of restriction enzyme B (A-B fragment), and the Y coordinate reflects the distance from the recognition site of restriction enzyme A to that of restriction enzyme C (A-C fragment) (Figure 1(6)). Accordingly, mutated points of a genome can be specified and mutation by DNA modification can be detected by analyzing the spot pattern. For example, since the density of a spot reflects the copy number of the detected fragments, it shows the duplication of specific regions, and where the position (X2, Y2) obtained after two-dimensional fraction in the spot pattern of figure 1(6) deviates or disappears, it shows that a portion of the nucleotide sequence of a DNA fragment is mutated (deleted, substituted or added).

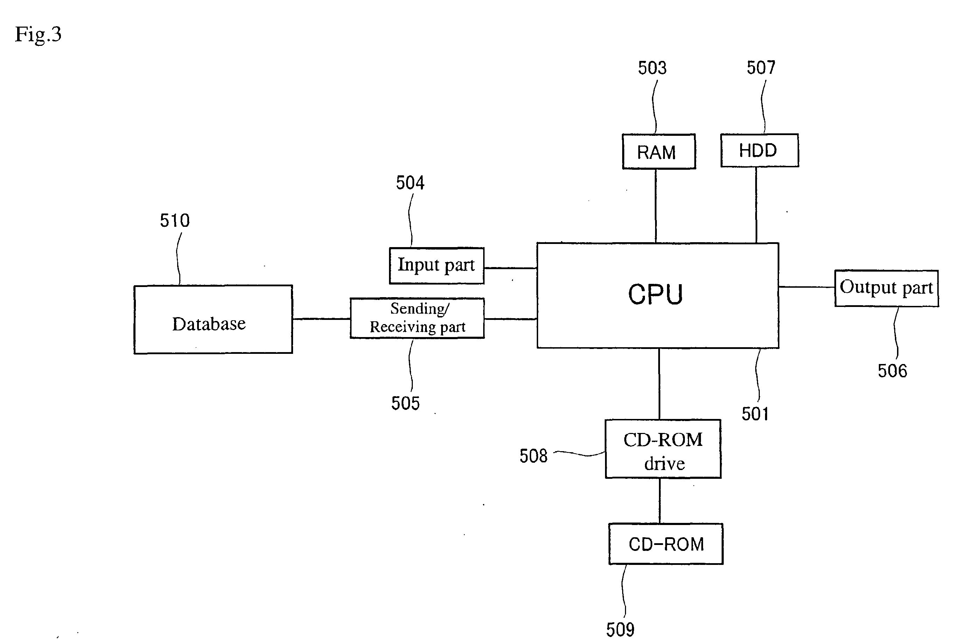

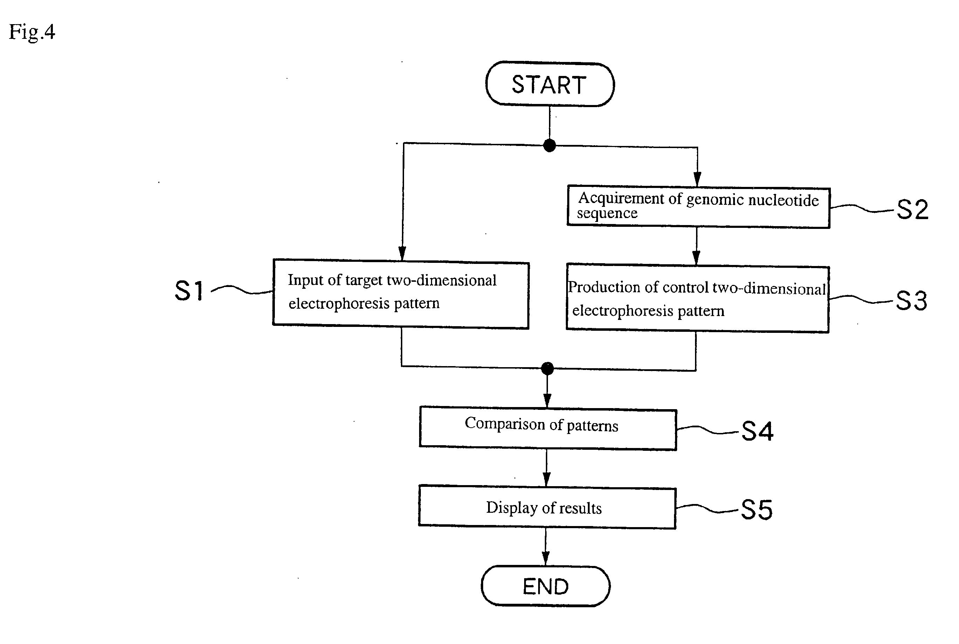

A program analyzing the two-dimensional electrophoresis pattern of the target genomic DNA obtained as stated above is described below. Figure 3 is a block diagram showing a system configuration including a computer device which executes this program. Figure 4 is a flow chart showing the concrete processing of the program using this system.

A computer executing this program is equipped with CPU 501 which controls the operation as a whole, RAM 503 which temporarily stores necessary program data and the like, input portion 504 into which an operator inputs various information, sending/receiving portion 505 which comprises a communication modem sending/receiving data to/from outside via the internet, output portion 505 which outputs data to be displayed, HDD 507 which records data and program etc., and CD drive 508 which reads data written onto CD-ROM 509. Furthermore, this system comprises database 510 which is connected to the computer by means of a communication line network such as the internet. This program can be recorded in any recording medium, as long as it is an information recording medium which is generally used as an external memory device of a computer, such as a tape-form or disk-form magnetic recording medium or an optical recording medium etc.

According to a program recorded into RAM 503, HDD 507 or CD-ROM 509, CPU 501 controls the whole system and executes a process described later. Input portion 504 may be a scanner, a mouse and a key board etc., and this is operated when inputting conditions necessary for executing a processing. Output portion 506 may be a display and a printer etc., and this is able to display data and the like processed under the control of CPU 501. Sending/receiving portion 505 is able to take data which was recorded in database 510 into RAM 503 and HDD 507 etc. under the control of CPU 501.

In this process, as shown in Figure 4, step S1 may be performed simultaneously with step S2 and step S3. In this process, step S1 may be performed prior to steps S2 and S3, or steps S2 and S3 may be performed prior to step S1.

In step S1, as stated above, the two-dimensional pattern obtained from a target genomic DNA is input. Specifically, each spot position (Xn, Yn) obtained in "(7) Detection of spots" can be input as digital data. Furthermore, a two-dimensional electrophoresis pattern can be input as image data using a scanner etc.

In step S2, genomic nucleotide sequence information recorded in database 510 is obtained by means of a communication line network such as the internet. This genomic nucleotide sequence information is a nucleotide sequence which is registered as a wild type of a target genomic DNA. Examples of database 510 include Genbank, EMBL, DDBJ, TAIR and KAOS etc. The genomic nucleotide sequence information used for this program is not limited to that recorded in database 510, but those recorded in HDD 507, CD-ROM 509 and other information recording mediums etc. can also be used.

In step S2, abnormal information comprised in the genomic nucleotide sequence information is preferably detected. Specifically, in step S2, abnormal information comprised in the genomic nucleotide sequence information is detected and temporarily stored, for example, in RAM 503 as a sorting.

The term "abnormal information" used herein means that nucleotides located in a certain position are unidentified, and such information is comprised in a nucleotide sequence, for example, as literal information such as "m", "r", "w", "s", "y", "k", "v", "h", "d", "b" and "n" etc. The character "m" represents that the position is A or C. The character "r" represents that the position is G or A. The character "w" represents that the position is A, T or U. The character "s" represents that the position is G or C. The character "y" represents that the position is T, U or C. The character "k" represents that the position is G, T or U. The character "v" represents that the position is A, G or C. The character "h" represents that the position is A, C, T or U. The character "d" represents that the position is A, G, T or U. The character "b" represents that the position is G, C, T or U. The character "n" represents that the position is A, G, C, T or U, or unknown.

In step S3, using the genomic nucleotide sequence information obtained from database 510, a control two-dimensional electrophoresis pattern is produced. When the control two-dimensional electrophoresis pattern is produced, first, the recognition sequence of restriction enzyme A and that of restriction enzyme B are retrieved from genomic nucleotide sequence information in order to predict A-A fragments and A-B fragments. Then, where an adapter is used for a target two-dimensional electrophoresis pattern, the nucleotide sequence number of the adapter is added to estimated A-A fragments and A-B fragments. According to this, there can be estimated a plurality of estimated A-A fragments and estimated A-B fragments corresponding to a plurality of A-A fragments and A-B fragments obtained by "(3) Treatment for genomic DNA with a second restriction enzyme".

Subsequently, based on the nucleotide sequence number of a plurality of estimated A-A fragments and estimated A-B fragments and estimated isoelectric points etc., an electrophoresis pattern, which is presumably obtained when "(4) First-dimension fractionation" is performed, is estimated. This electrophoresis pattern can be calculated by the method reported by Southern, E. M. (Measurement of DNA Length by Gel Electrophoresis, *Analytical Biochemistry:* 100, 319-323, 1979). Furthermore, the electrophoresis pattern can be estimated by inputting, as parameters, various conditions such as the concentration and type of gel, a voltage under which an electrophoresis is performed, electrophoresis period and temperature etc. In other words, in the present program, a correction value is preferably provided in advance so that an electrophoresis pattern can be estimated using these conditions as parameters.

The recognition sequence of restriction enzyme C in estimated A-A fragments and estimated A-B fragments is retrieved, and A-C fragments are estimated. According to this process, a plurality of estimated A-C fragments corresponding to a plurality of A-C fragments obtained in "(5) Treatment for genomic DNA with a third restriction enzyme" can be estimated.

Then, based on the nucleotide sequence number of a plurality of estimated A-C fragments and estimated isoelectric points etc., an electrophoresis pattern, which is presumably obtained when "(6) Second-dimension fractionation" is performed, is estimated. Just as in the estimation of "electrophoresis pattern" stated above, this electrophoresis pattern can be estimated by inputting, as parameters, various conditions such as the type of gel, a voltage under which an electrophoresis is performed, electrophoresis period and temperature etc. In other words, in the present program, a correction value is preferably provided in advance so that an electrophoresis pattern can be estimated using these conditions as parameters.

In the present program, based on the obtained control two-dimensional electrophoresis pattern, an estimated spot position can be obtained as an estimated coordinate (Xn, Yn). Herein, X coordinate (Xn) represents a size-fraction position of A-B fragments to which the nucleotide sequence number of an adapter is added, and Y coordinate (Yn) represents a size-fraction position of A-C fragments to which the nucleotide sequence number of an adapter is added. In this step S3, a control two-dimensional electrophoresis pattern can also be obtained as image data based on the obtained estimated coordinates (Xn, Yn).

In the above description, an electrophoresis pattern is produced based on estimated A-B fragments. However, where "(3) Second treatment with a second restriction enzyme" is not performed when a target two-dimensional electrophoresis pattern is produced, the electrophoresis pattern may be produced based on estimated A fragments which are estimated to be obtained by treatment with restriction enzyme A. Where an adapter is not added when a target two-dimensional electrophoresis pattern is produced, the nucleotide sequence number of the adapter may not be considered.

Moreover, a control two-dimensional electrophoresis pattern is preferably produced as one which excludes a certain region from genomic nucleotide sequence information. Examples of regions to be excluded include a telomere region of genomic DNA and high frequency repeated nucleotide sequences located around a centromere of genomic DNA etc. Specifically, before performing treatment with a third restriction enzyme, fragments having the recognition sequence of restriction enzyme A only at one terminus thereof are not identified as spots, as they are derived from a telomere region of genomic DNA or a gap region during nucleotide sequencing.

In the present method, where abnormal information comprised in the genomic nucleotide sequence information is detected in step S2, a recognition sequence such as restriction enzyme A is detected, setting genomic nucleotide sequence information other than the abnormal information as a target. That is, a nucleotide which is located in a position detected as abnormal information is eliminated from a target, when detecting a recognition sequence such as restriction enzyme A.

Moreover, in the present method, where abnormal information comprised in the genomic nucleotide sequence information is detected in step S2, the abnormal information is linked to an estimated spot. Specifically, abnormal information such as whether or not abnormal information is found in a DNA fragment comprised in an estimated spot, and the type of abnormal information comprised in an estimated spot, is linked to each of the spots.

In step S4, the control two-dimensional electrophoresis pattern obtained based on genomic nucleotide sequence information is compared with a target two-dimensional electrophoresis pattern. Specifically, a coordinate (Xn, Yn) of a spot input in step S is compared with an estimated coordinate (Xn, Yn) of the spot obtained in step S3. Furthermore, in step S4, it is also possible that the image data of the two-dimensional electrophoresis pattern obtained in step S is compared with that obtained in step S3.

In step S5, the results of comparison performed in step S4 are displayed on a display. Such comparison results are provided by identifying spots in a control two-dimensional electrophoresis pattern, which do not match with a target two-dimensional electrophoresis pattern, and display the coordinates of these spots and nucleotide sequence information thereof. Since a control two-dimensional electrophoresis pattern is produced based on the genomic nucleotide sequence information obtained from database 510, the nucleotide sequence information can correspond to each of the spots.

Especially when cells having a chromosomal structure such as that of higher organisms are used as targets for analysis, if a telomere region of genomic DNA or a high frequency repeated nucleotide sequence located around a centromere which shows unclear data during nucleotide sequencing, is used as a control, it causes noise. Thus, the comparison of spots causing noise can be prevented by using a control two-dimensional electrophoresis pattern, from which a telomere region of genomic DNA and a high frequency repeated nucleotide sequence located around a centromere of genomic DNA are removed. At the initial stage, both ends of each clone are marked at the initial stage to clearly distinguishing noise appearing in a pattern. By these processes, a comparison with higher accuracy can be performed, having decreased noise components.

In step S5, where abnormal information comprised in the genomic nucleotide sequence information is detected in step S2, there is identified a spot in a control two-dimensional electrophoresis pattern, which does not match with a target two-dimensional electrophoresis pattern, and determined whether the spot comprises abnormal information or not. As a result, where abnormal information is comprised in the identified spot, the abnormal information is corrected and the control two-dimensional electrophoresis pattern is reconstructed. For example, where abnormal information "m" is comprised in the identified spot, there are reconstructed both a control two-dimensional electrophoresis pattern in which the position of abnormal information is set at A, and another control two-dimensional electrophoresis pattern in which the position of abnormal information is set at C.

Then, in step S5, the thus reconstructed control two-dimensional electrophoresis patterns are compared with a target two-dimensional electrophoresis pattern again. For example, where abnormal information "m" is comprised in the identified spot, and both control two-dimensional electrophoresis patterns in which the positions of abnormal information are set at A or C were reconstructed, these two types of control two-dimensional electrophoresis patterns are compared with a target two-dimensional electrophoresis pattern.

According to this step, the analysis of a target two-dimensional electrophoresis pattern can be carried out with higher precision, while reflecting abnormal information in genomic nucleotide sequence information. Where it is impossible to correct the obtained abnormal information, information regarding abnormal information comprised in the identified spot is linked to the spot.

As described above, according to the program of the present invention, a control two-dimensional electrophoresis pattern is produced using genomic nucleotide sequence information recorded in database 510 etc., and a target two-dimensional electrophoresis pattern is analyzed using this control two-dimensional electrophoresis pattern. Hence, according to this program, when a two-dimensional electrophoresis pattern obtained from genomic DNA is analyzed, it is not necessary to produce a control two-dimensional electrophoresis pattern using genomic DNA extracted from wild type cell strains. Moreover, according to this program, enormous manpower and complex techniques are also not needed. Therefore, using to this program, target genomic DNA can be analyzed easily and rapidly.

Especially in the present program, genomic DNA having a mutation such as a deletion of nucleotides induced by a physical mutagene is preferably used. That is to say, in the present program, it is preferable to use as a target, genomic DNA the length of which differs from a wild type genomic DNA. Specifically, mutation-introduced genomic DNA is used as a target, applying a means involving induction of mutation by radiation of heavy ion beam (Japanese Patent Application Laying-Open (kokai) No. 9-28220).

Particularly where genomic DNA of plant cells is analyzed by applying the present program, since cytosine existing in plant genomic DNA is often methylated, methylation insensitive restriction enzymes are preferably used as the first restriction enzymes. That is, where plant genomic DNA is used as a target, using methylation insensitive restriction enzymes, the DNA is reliably cleaved under no influence of methylation. Examples of methylation insensitive restriction enzymes used for the first restriction enzyme treatment include *Acc*III and *Pac*I etc. Examples of methylation insensitive restriction enzymes used for the second restriction enzyme treatment include *Mbo*I recognizing 4 nucleotides. Using these restriction enzymes, even where plant genomic DNA is used as a target, many spots can be obtained under no influence of methylation, thereby accomplishing more precise analysis.

Furthermore, where genomic DNA is analyzed, methylation sensitive restriction enzymes may also be used. In this case, if methylation sensitive restriction enzymes are used as the first restriction enzymes, epigenetic mutated regions caused by methylation of DNA can be detected and analyzed.

### EXAMPLES

The present invention is further described in the following examples. The examples are provided for illustrative purposes only, and are not intended to limit the scope of the invention.

### Example 1.

### Arabidopsis thaliana chloroplast DNA

### [Production of control two-dimensional electrophoresis pattern]

In this example, *Arabidopsis thaliana* chloroplast DNA is used as an analysis target.

The nucleotide sequence of chloroplast DNA of *Arabidopsis thaliana* (Columbia-strain) has already been determined as one having a circular genomic DNA of 154,478bp (Sato, S. *et al*. 1999, *DNA RESEARCH* 6, 238-290), and the nucleotide sequence data can be obtained from DDBJ as Accession No. AP000423. Based on this nucleotide sequence data, a control two-dimensional electrophoresis pattern was produced by the above-stated program processes.

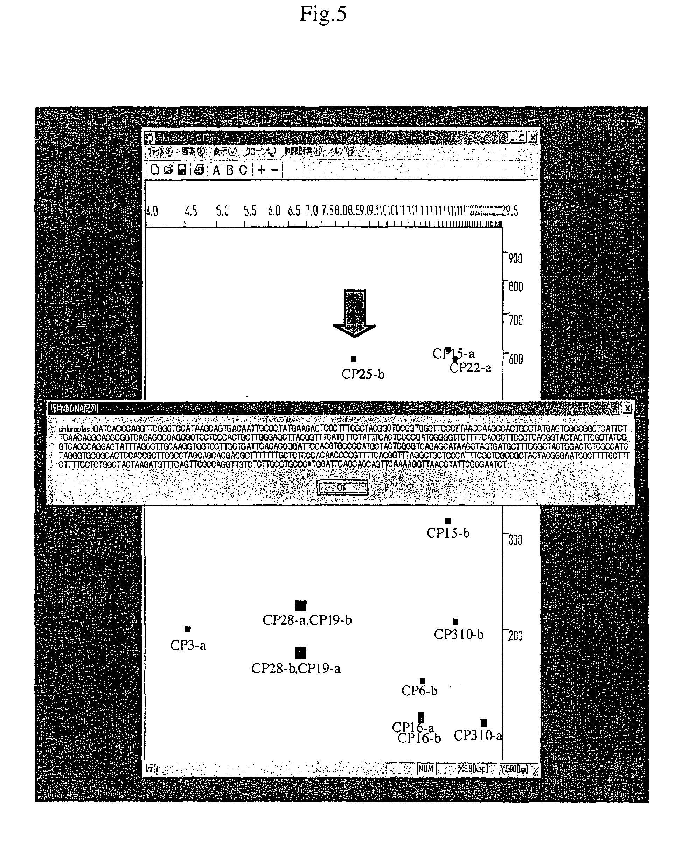

In this example, a two-dimensional electrophoresis pattern was produced, using *Acc*III as the first restriction enzyme and *Mbo*I as the third restriction enzyme but not using the second restriction enzyme. These *Acc*III and *Mbo*I are insensitive to 5-methyl cytosine (5mC) which results from methylation of cytosine. The obtained control two-dimensional electrophoresis pattern is shown in Figure 5. In the figure, the display of nucleotide number shows a mobility when a molecular weight marker was actually electrophoresed, and the horizontal axis shows development of a first-dimension fractionation and the longitudinal axis shows development of a second-dimension fractionation.

The nucleotide sequence of spot CP3-a in the control two-dimensional electrophoresis pattern (Figure 5) produced in this example is shown in SEQ ID NO: 2, the nucleotide sequence of spot CP6-b is shown in SEQ ID NO: 3, the nucleotide sequence of spot CP16-a is shown in SEQ ID NO: 4, the nucleotide sequence of spot CP16-b is shown in SEQ ID NO: 5, the nucleotide sequence of spot CP19-a is shown in SEQ ID NO: 6, the nucleotide sequence of spot CP19-b is shown in SEQ ID NO: 7, the nucleotide sequence of spot CP22-a is shown in SEQ ID NO: 8, the nucleotide sequence of spot CP25-b is shown in SEQ ID NO: 9, the nucleotide sequence of spot CP28-a is shown in SEQ ID NO: 10, the nucleotide sequence of spot CP28-b is shown in SEQ ID NO: 11, the nucleotide sequence of spot CP310-a is shown in SEQ ID NO: 12, the nucleotide sequence of spot CP310-b is shown in SEQ ID NO: 13, the nucleotide sequence of spot CP15-a is shown in SEQ ID NO: 14, and the nucleotide sequence of spot CP15-b is shown in SEQ ID NO: 15.

When a spot is selected in a control two-dimensional electrophoresis pattern shown in Figure 5, there is displayed the nucleotide sequence of a DNA fragment comprised in the selected spot. Figure 5 shows the nucleotide sequence of a DNA fragment comprised in the selected spot CP25-b.

### [Production of target two-dimensional electrophoresis pattern]

Setting chloroplast DNA of *Arabidopsis thaliana* (Columbia-strain) as an analysis target, a two-dimensional electrophoresis pattern was actually produced as follows, using *Acc*III and *Mbo*I. First, the extracted chloroplast DNA was digested with *Acc*III, followed by label reaction with [α-³²P]dCTP or [α-³²P]dGTP for 30 minutes, using sequenase. After that, 0.8% agarose gel electrophoresis was performed at 2V/cm at room temperature (24°C) for 48 hours. Further, for a third restriction enzyme treatment to the gel, 500 units of *Mbo*I per tube was applied at 37°C for 2 hours. Then, a second-dimension fractionation was performed by 5% acrylamide electrophoresis. Conditions of the electrophoresis were set at 2V/cm, at room temperature (24°C), for 22 hours.

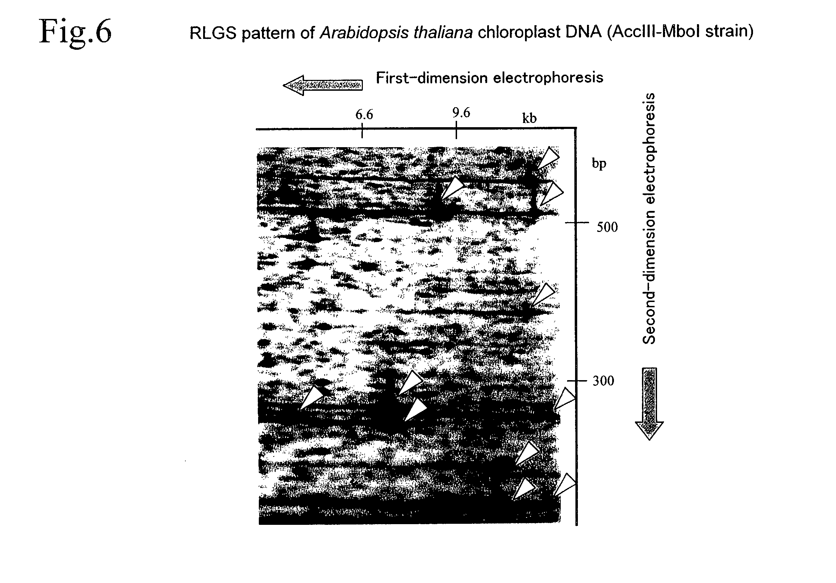

A two-dimensional electrophoresis pattern was obtained by drying the gel, performing exposures at -80°C for 14 days, and developing. A two-dimensional pattern obtained by analyzing chloroplast DNA is shown in Figure 6. In the figure, open triangles show the positions of spots observed.

### [Identification of spots by comparison of both patterns]

A comparison was made between a control two-dimensional electrophoresis pattern shown in Figure 5 and a target two-dimensional electrophoresis pattern shown in Figure 6. As clearly shown in Figures 5 and 6, the positions of spots in both two-dimensional electrophoresis patterns almost matched.

Among spots shown in Figure 6, DNA fragments were extracted from spots corresponding to estimated spots, CP3-a (SEQ ID NO: 2) and CP15-b (SEQ ID NO: 15) shown in Figure 5, and were cloned to determine each of the nucleotide sequences. As a result, each of the obtained nucleotide sequences completely matched nucleotide sequence information for each of CP3-a and CP15-b in genome sequence information obtained from a database.

This result clearly shows that an actual target two-dimensional electrophoresis pattern could be analyzed, using a control two-dimensional electrophoresis pattern obtained based on genomic nucleotide sequence information.

Moreover, the target two-dimensional electrophoresis pattern shown in Figure 6 shows that a spot indicates a high signal strength when chloroplast DNA is used. This is because the copy numbers of chloroplast DNA are numerous. From this result, it was shown that the greater the amount of target genomic DNA, the higher the signal strength that can be displayed.

### Example 2.

### Arabidopsis thaliana nuclear genomic DNA

### [Production of control two-dimensional electrophoresis pattern]

In this example, *Arabidopsis thaliana* nuclear genomic DNA is used as an analysis target. In respect of nuclear genomic DNA of *Arabidopsis thaliana* (Columbia-strain), although partial gap regions (an undetermined region) still remains, almost the entire nucleotide sequence, about 130Mb, has already been determined (refer to The Arabidopsis Genome Initiative, *Nature*., 408 796-815 (2000), which has been disclosed in The Arabidopsis Information resource (referred to as TAIR, www.arabidopsis.org/some.html)). Based on this nucleotide sequence data, a control two-dimensional electrophoresis pattern was produced by the above-stated program processes.

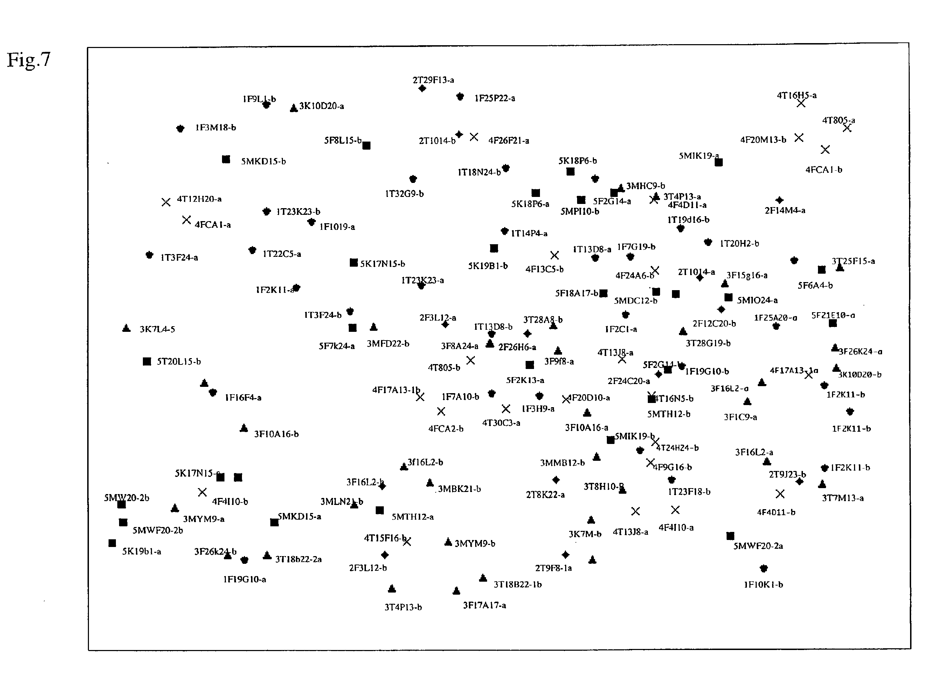

In this example, a two-dimensional electrophoresis pattern was produced, using *Not*I as the first restriction enzyme, *Eco*RV as the second restriction enzyme and *Mbo*I as the third restriction enzyme. These *Eco*RV and *Mbo*I are insensitive to 5-methyl cytosine (5mC) which results from methylation of cytosine, but *Not*I is sensitive. The obtained control two-dimensional electrophoresis pattern is shown in Figure 7. In the figure, the display of nucleotide number shows mobility when a molecular weight marker was actually electrophoresed, and the horizontal axis shows development of a first-dimension fractionation and the longitudinal axis shows development of a second-dimension fractionation. Furthermore, in Figure 7, the character string described corresponding to each spot shows the name of gene locus comprising *Not*I site in genomic DNA. Each number from 1 to 5 located at the extreme left denotes chromosome number, and either "-a" or "-b" located at the extreme right denotes respectively the upstream or downstream side of *Not*I site. Character strings other than above stated denote the names of clones which were produced during determination of nucleotide sequences. For example, a spot to which "4FCA1-b" is linked is derived from chromosome number 4, and it means a DNA fragment having a determined nucleotide sequence, comprising a DNA fragment being downstream of *Not*I site in FCA1 clone. In Figure 7, a black circle (●) means a spot derived from chromosome number 1, a black rhombus (◆) means a spot derived from chromosome number 2, a black triangle (▲) means a spot derived from chromosome number 3, a cross (×) means a spot derived from chromosome number 4, and a black square (■) means a spot derived from chromosome number 5.

### [Production of target two-dimensional electrophoresis pattern]

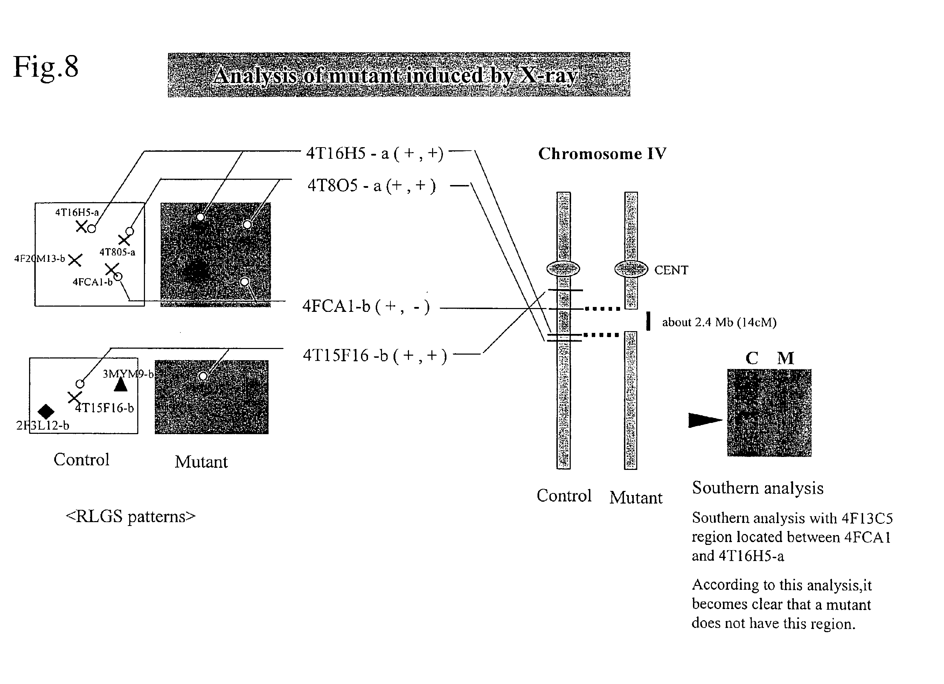

Setting, as an analysis target, genomic DNA of *Arabidopsis thaliana* (Columbia-strain) to which a mutation was induced by X-ray irradiation, a two-dimensional electrophoresis pattern was actually produced by the same process as in Example 1 with the exception that *Not*I was used as the first restriction enzyme, *Eco*RV as the second restriction enzyme and *Mbo*I as the third restriction enzyme. The obtained two-dimensional pattern is shown as "Mutant" in Figure 8.

In Figure 8, "Control" means a portion excerpted in part from a control two-dimensional electrophoresis pattern and enlarged. When comparing "Mutant" with "Control", it was confirmed that 4FCA1-b disappeared as a result of X-ray irradiation. Furthermore, when the presence or absence of spots of *Not*I sites located on both sides of 4FCA1-b was examined, spots of 4T15F16-b being upstream, and 4T16H5-a and 4T805-a being downstream could be confirmed, thereby suggesting that a deletion of at most about 2.4Mb occurred between 4FCA1-b and 4T16H5-a. To verify this deletion, Southern Analysis was carried out using, as a probe, a portion of 4F13C5 region located between 4FCA and 4T16H5. As a result, as shown in "Southern analysis" in Figure 8, deletion of this region could be confirmed. In "Southern analysis" in the figure, C lane is a sample which was not subjected to X-ray irradiation and M lane is a sample which was subjected to X-ray irradiation.

Thus, using a control two-dimensional electrophoresis pattern shown in Figure 7, it was clarified that a mutated region in a mutant can be detected. That is to say, it was clarified that a mutated region in a mutant can be efficiently detected in a very short time by comparing a target two-dimensional electrophoresis pattern and a control two-dimensional electrophoresis pattern.

In the obtained two-dimensional electrophoresis pattern ("Mutant" in Figure 8), some spots found in the two-dimensional electrophoresis pattern shown in Figure 7 could not be found. This is because *Not*I is sensitive to methylation and spots do not appear under the influence of methylation in chromosomal DNA. Where low methylation of chromosomal DNA is achieved by demethylation, these spots can be observed.

Therefore, it was clarified that, using a control two-dimensional electrophoresis pattern shown in Figure 7, a mutated region in a mutant cannot only be detected, but DNA modification such as methylation of DNA can also be detected.

### [Detection of abnormal information]

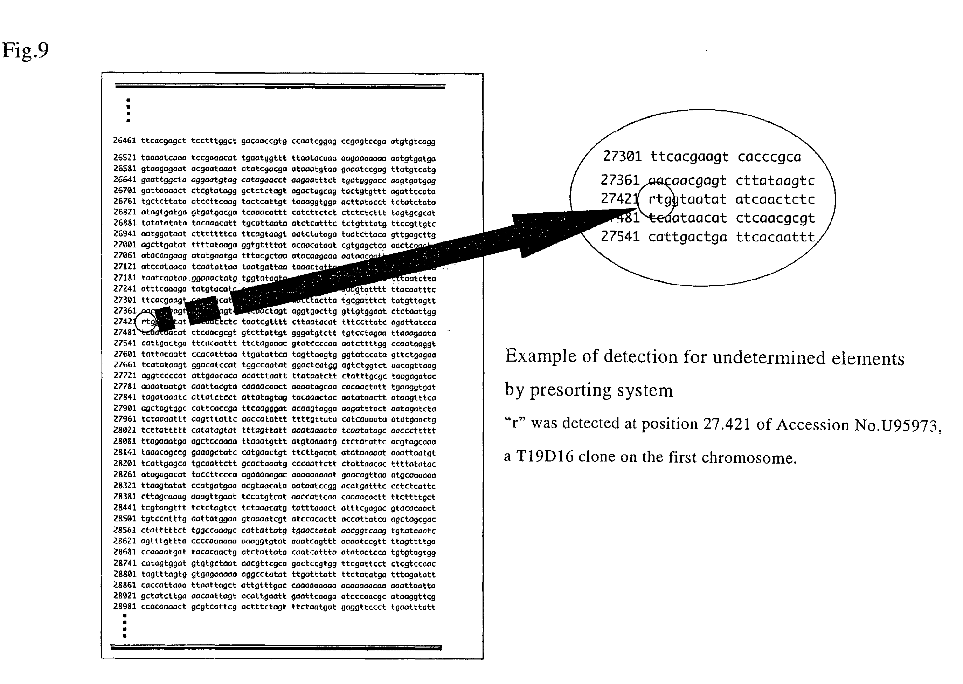

In this example, when the above-described "production of control two-dimensional electrophoresis pattern" is performed, first, abnormal information in the nucleotide sequence information regarding *Arabidopsis thaliana* nuclear genome was detected. Specifically, character strings other than A, G, C and T comprised in the nucleotide sequence information are detected, and the extracted character strings other than A, G, C and T are defined as abnormal information. As a result, as an example shown in Figure 9, abnormal information "r" was detected at position 27,421 of Accession No. U95973 (detected in DDBJ: http://ftp2.ddbj.nig.ac.jp: 8000/getstart-j.html), a T19D16 clone on the first chromosome.

This case means that abnormal information is comprised in a spot derived from a T19D16 clone, in a control two-dimensional electrophoresis pattern shown in Figure 7. Accordingly, in advance of performing a comparison between the control two-dimensional electrophoresis pattern shown in Figure 7 and a target two-dimensional electrophoresis pattern, a spot derived from a T19D16 clone is linked to the abnormal information. According to this step, where a mismatch was observed regarding a spot derived from the T19D16 clone in the above comparison, it shows that abnormal information is linked to the spot derived from the T19D16 clone.

Then, it is determined that the mismatch regarding a spot derived from a T19D16 clone is caused by abnormal information or not. The determination is carried out by correcting abnormal information "r" to "G" or "A" to prepare 2 types of control two-dimensional electrophoresis patterns, and comparing each of these 2 types of control two-dimensional electrophoresis pattern with a target two-dimensional electrophoresis pattern. Where a spot derived from a T19D16 clone in the target two-dimensional electrophoresis pattern matches with that in either of the 2 control two-dimensional electrophoresis patterns, it shows that the mismatch is caused by abnormal information.

Thus, a target two-dimensional electrophoresis pattern was able to be analyzed more in detail by detecting abnormal information in the nucleotide sequence information regarding *Arabidopsis thaliana* nuclear genome and linking the abnormal information to a spot in a control two-dimensional electrophoresis pattern.

### Effect of the Invention

As stated in detail above, according to the present invention, a control two-dimensional electrophoresis pattern to analyze a target genomic DNA can easily be produced under various conditions. Therefore, the present invention provides a program for easily analyzing a two-dimensional electrophoresis pattern produced using genomic DNA.

Furthermore, the present invention provides a method for quickly and reliably analyzing genomic DNA by producing a control two-dimensional electrophoresis pattern based on genomic nucleotide sequence information.

## Claims

1. A program which allows a computer to execute the processes of:
producing a control two-dimensional electrophoresis pattern based on genomic nucleotide sequence information,
comparing said control two-dimensional electrophoresis pattern and a target two-dimensional electrophoresis pattern obtained by performing two-dimensional electrophoresis using target genomic DNA, and
detecting a difference in spot position between said control two-dimensional electrophoresis pattern and said target two-dimensional electrophoresis pattern.

2. The program according to claim 1, wherein, in said process of producing the control two-dimensional electrophoresis pattern, the control two-dimensional electrophoresis pattern is produced by detecting the recognition sequence of a first restriction enzyme and the recognition sequence of a second restriction enzyme in said genomic nucleotide sequence information, and basing on a nucleotide sequence flanked by the first restriction enzyme recognition sequence and the second restriction enzyme recognition sequence and another nucleotide sequence flanked by the two first restriction enzyme recognition sequences.

3. The program according to claim 2, wherein said first restriction enzyme and said second restriction enzyme are selected from methylation insensitive restriction enzymes.

4. The program according to claim 2, wherein said first restriction enzyme and said second restriction enzyme are selected from methylation sensitive restriction enzymes.

5. The program according to any one of the preceding claims, which comprises a process of obtaining said genomic nucleotide sequence information by means of a communication line network.

6. The program according to any one of the preceding claims, wherein, in said process of producing the control two-dimensional electrophoresis pattern, a plurality of spots are produced based on genomic nucleotide sequence information and these spots are linked to gene loci information on a genome.

7. The program according to any one of the preceding claims, wherein, in said process of producing the control two-dimensional electrophoresis pattern based on a genomic nucleotide sequence information, abnormal information comprised in said genomic nucleotide sequence information is detected.

8. The program according to claim 7, which links the detected abnormal information to a spot comprised in the produced two-dimensional electrophoresis pattern in order to memorize the information.

9. A method for analyzing genomic DNA which comprises the steps of:
producing a two-dimensional electrophoresis pattern by performing two-dimensional electrophoresis using a target genomic DNA,
comparing said two-dimensional electrophoresis pattern and a control two-dimensional electrophoresis pattern produced based on genomic nucleotide sequence information, and
detecting a difference in spot position between said control two-dimensional electrophoresis pattern and said target two-dimensional electrophoresis pattern.

10. The method for analyzing genomic DNA according to claim 9, which comprises a step of extracting said target genomic DNA from plant cells.

11. The method for analyzing genomic DNA according to claim 9, wherein said target genomic DNA is derived from a higher organism.

12. The method for analyzing genomic DNA according to any one of claims 9 to 11, wherein, in said step of producing the two-dimensional electrophoresis pattern of said target genomic DNA by the following steps (a) to (e):
(a) treating genomic DNA with a first restriction enzyme,
(b) adding a label to a site cleaved with said restriction enzyme,
(c) performing a first-dimension fractionation on the obtained DNA fragment by electrophoresis,
(d) after treating the DNA fragment fractioned in step (c) with a second restriction enzyme, performing a second-dimension fractionation, and
(e) detecting a spot of the labeled DNA fragment fractioned in step (d).

13. The method for analyzing genomic DNA according to claim 12, wherein, after said step (b), the obtained DNA fragment is treated with a restriction enzyme different from the first and second restriction enzymes and before said step (c) is performed.

14. The method for analyzing genomic DNA according to claim 12 or claim 13, which comprises detecting the recognition sequences of said first and second restriction enzymes in genomic nucleotide sequence information, and producing the control two-dimensional electrophoresis pattern based on these cleavage sites.

15. The method for analyzing genomic DNA according to any one of claims 12 to 14, wherein said step (b) is carried out by connecting one end of an adapter to the restriction enzyme cleavage site as well as adding said label to the other end of said adapter.

16. The method for analyzing genomic DNA according to any one of claims 9 to 15, wherein said control two-dimensional electrophoresis pattern has a plurality of spots produced based on genomic nucleotide sequence information and links these spots to gene loci information on a genome.

17. The method for analyzing genomic DNA according to any one of claims 9 to 16, which comprises detecting abnormal information comprised in genomic nucleotide sequence information before producing the control two-dimensional electrophoresis pattern based on said genomic nucleotide sequence information.

18. The method for analyzing genomic DNA according to claim 17, which comprises linking the detected abnormal information to a spot comprised in the produced two-dimensional electrophoresis pattern.

19. A method for identifying genes associated with mutant genes and/or mutant traits, which comprises detecting mutated sites in target genomic DNA by the method for analyzing genomic DNA according to any one of claims 9 to 18.

20. A method for isolating genes associated with mutant genes and/or mutant traits, which comprises isolating a DNA fragment containing mutated sites detected by the identification method according to claim 19, from said two-dimensional electrophoresis pattern.

21. A two-dimensional electrophoresis pattern, which has a plurality of spots produced based on genomic nucleotide sequence information and estimated from said genomic nucleotide sequence information.

22. The two-dimensional electrophoresis pattern according to claim 21, wherein said plurality of spots are linked to gene loci information on a genome.

23. The two-dimensional electrophoresis pattern according to claim 21, wherein said genomic nucleotide sequence information is nucoleotide sequence information obtained from a plant nuclear genome.

24. The two-dimensional electrophoresis pattern according to claim 21, wherein abnormal information detected from said genomic nucleotide sequence information is linked to said plurality of spots.

25. The two-dimensional electrophoresis pattern according to claim 21, wherein said genomic nucleotide sequence information is obtained from the genome of a higher organism.


SEQUENCE LISTING

<110> RIKEN

<120> Genomic DNA Analysis Program

<130> AHB/FP5964788

<140> EP
<141>

<150> JP2000-327632
<151> 2000-10-26

<150> JP2001-84370
<151> 2001-03-23

<160> 15

<170> PatentIn Ver. 2.0

<210> 1
<211> 12
<212> DNA
<213> Artificial Sequence

<220>
<223> Description of Artificial Sequence:Synthetic DNA

<220>
<221> modified base
<222> 4, 5, 6, 7, 8 and 9
<223> n represents a, g, c or t

<400> 1
ccannnnnnt gg                                                     12

<210> 2
<211> 207
<212> DNA
<213> Arabidopsis thaliana (Columbia)

<400> 2
tccggataat atccataaat gacttgtttt tggtaagagg tggacattac tatatgtaaa 60
ttcgggtgca tgggacacat cagtactcca gtgcatttcg ccctctgagt cagaataaat 120
atattttcta accctctctt taaaatgaaa agtgtatgtt ccctcgcgaa tctcagcaat 180
cacttgttct gattccacat attgatc                                     207

<210> 3
<211> 163
<212> DNA
<213> Arabidopsis thaliana (Columbia)

<400> 3
gatcttttat actgtgtccg attccaaagt tcgttctata catatgaccc gcaatgagga 60
aaagaattgc gatagctaaa tgatgatgtg ccatatcggt tagccataaa ctttgcgttt 120
gtggatggaa tcccccaaga agggttagaa tggcagttcc gga                   163

<210> 4
<211> 131
<212> DNA

<213> Arabidopsis thaliana (Columbia)

<400> 4
tccggaatat gagtgtgtga cttgttagaa ttgaccctat ggatagtaca gagaatgggg 60
tctgtcatct ttatcaagat ggttttactt cgtcggatat tcattcgagt atctggagca 120
cgaaatagat c 131

<210> 5
<211> 134
<212> DNA
<213> Arabidopsis thaliana (Columbia)

<400> 5
gatccttcct ttattcaaac ggaaggaaga gagatagaat cagaccgatt ccctaaatac 60
ctttctggct attcctcaat gccccggcta ttcacggaac gtgagaagcg aatgaataat 120
catctgcttc cgga 134

<210> 6
<211> 189
<212> DNA
<213> Arabidopsis thaliana (Columbia)

<400> 6
tccggaggat gccttatata tatattaata tatattatat caaaaagatg gacaatcaaa 60
tctatttctc gattcaatag aagtccaacc aaagaggtga atagggtccc aaataacgag 120
agatatgtaa aaagtaggtc agatttcgcc tattcctaat cctaaatgga atgtaacgac 180
gtagggatc 189

<210> 7
<211> 232
<212> DNA
<213> Arabidopsis thaliana (Columbia)

<400> 7
gatcagccac actgggactg agacacggcc cagactccta cgggaggcag cagtggggaa 60
ttttccgcaa tgggcgaaag cctgacggag caatgccgcg tggaggtaga aggcctacgg 120
gtcctgaact tcttttccca gagaagaagc aatgacggta tctggggaat aagcatcggc 180
taactctgtg ccagcagccg cggtaataca gaggatgcaa gcgttatccg ga 232

<210> 8
<211> 597
<212> DNA
<213> Arabidopsis thaliana (Columbia)

<400> 8
tccggagatt cccgaatagg ttaacctttt gaactgctgc tgaatccatg ggcaggcaag 60
agacaacctg gcgaactgaa acatcttagt agccagagga aaagaaagca aaagcgattc 120
ccgtagtagc ggcgagcgaa atgggagcag cctaaaccgt gaaaacgggg ttgtgggaga 180
gcaaaaaaag cgtcgtgctg ctaggcgaag cggtggagtg ccgcacccta gatggcgaga 240
gtccagtagc cgaaagcatc actagcttat gctctgaccc gagtagcatg gggcacgtgg 300
aatcccgtgt gaatcagcaa ggaccacctt gcaaggctaa atactcctgg gtgaccgata 360
gcgaagtagt accgtgaggg aagggtgaaa agaaccccca tcggggagtg aaatagaaca 420
tgaaaccgta agctcccaag cagtgggagg agccctgggc tctgaccgcg tgcctgttga 480
agaatgagcc ggcgactcat aggcagtggc ttggttaagg gaacccaccg gagccgtagc 540
gaaagcgagt cttcataggg caattgtcac tgcttatgga cccgaacctg ggtgatc 597

<210> 9
<211> 592
<212> DNA
<213> Arabidopsis thaliana (Columbia)

<400> 9
gatcacccag gttcgggtcc ataagcagtg acaattgccc tatgaagact cgctttcgct 60
acggctccgg tgggttccct taaccaagcc actgcctatg agtcgccggc tcattcttca 120
acaggcacgc ggtcagagcc cagggctcct cccactgctt gggagcttac ggtttcatgt 180
tctatttcac tccccgatgg gggttctttt cacccttccc tcacggtact acttcgctat 240
cggtcaccca ggagtattta gccttgcaag gtggtccttg ctgattcaca cgggattcca 300
cgtgccccat gctactcggg tcagagcata agctagtgat gctttcggct actggactct 360
cgccatctag ggtgcggcac tccaccgctt cgcctagcag cacgacgctt tttttgctct 420
cccacaaccc cgttttcacg gtttaggctg ctcccatttc gctcgccgct actacgggaa 480
tcgcttttgc tttcttttcc tctggctact aagatgtttc agttcgccag gttgtctctt 540
gcctgcccat ggattcagca gcagttcaaa aggttaacct attcgggaat ct 592

<210> 10
<211> 232
<212> DNA
<213> Arabidopsis thaliana (Columbia)

<400> 10
tccggataac gcttgcatcc tctgtattac cgcggctgct ggcacagagt tagccgatgc 60
ttattcccca gataccgtca ttgcttcttc tctgggaaaa gaagttcagg acccgtaggc 120
cttctacctc cacgcggcat tgctccgtca ggctttcgcc cattgcggaa aattccccac 180
tgctgcctcc cgtaggagtc tgggccgtgt ctcagtccca gtgtggctga tc 232

<210> 11
<211> 190
<212> DNA
<213> Arabidopsis thaliana (Columbia)

<400> 11
gatccctacg tcgttacatt ccatttagga ttaggaatag gcgaaatctg acctactttt 60
tacatatctc tcgttatttg ggaccctatt cacctctttg gttggacttc tattgaatcg 120
agaaatagat ttgattgtcc atctttttga tataatatat attaatatat atataaggca 180
tccttccgga 190

<210> 12
<211> 134
<212> DNA
<213> Arabidopsis thaliana (Columbia)

<400> 12
tccggaagca gatgattatt cattcgcttc tcacgttccg tgaatagccg gggcattgag 60
gaatagccag aaaggtattt agggaatcgg tctgattcta tctctcttcc ttccgtttga 120
ataaaggaag gatc 134

<210> 13
<211> 215
<212> DNA
<213> Arabidopsis thaliana (Columbia)

<400> 13
gatcttgtgg gaagagtgtt gccaaattta tactgtccgc tatctcatag gtggctacgg 60
gccgcaccaa aacaaaaaac tttttcttgg ttggtgtaat ccgttggaca taaatccaat 120
ttttaaattt tttggattcc ttcgagtttt tttttcctct tcctggcggt atcaagatgc 180
cactgtgtcg ggatatttta tctgtcttgt ccgga 215

<210> 14
<211> 618
<212> DNA
<213> Arabidopsis thaliana (Columbia)

<400> 14

tccggaggag cacgaatgca agaaggaagt ttaagtttga tgcaaatggc taaaatttct 60
tcggttttat gtgattatca atcaagtaaa aagttattct atatatcaat tcttacatct 120
cctactaccg gtggagtgac agctagtttt ggtatgttgg gggatatcat tattgccgaa 180
ccctatgcct atattgcatt tgcgggtaaa agagtaattg aacaaacatt gaaaaaagcc 240
gtgcctgaag gttcacaagc ggctgaatct ttattacgta agggcttatt ggatgcaatt 300
gtaccacgta atcttttaaa aggtgttctg agcgagttat ttcagctcca tgcttttttt 360
cctttgaaca caaattaaat aaaatagaac ggttagttta tcagaattaa acgaaaaccc 420
agaaaaatgc atttttcttt caaatcattt ttttttatcg atattcttgt ttactactca 480
gtaaacctct atcaacaagc taaaaagtga atttttttgg gggggaagtt caaattagac 540
tagacaaaca aaaaaaagtt cattttcctc ccttgcttgc atatgtatag ataattcaaa 600
tatagataga tgcagatc 618

<210> 15
<211> 323
<212> DNA
<213> Arabidopsis thaliana (Columbia)

<400> 15
gatctggtcc aagaagcact actgtaggga agttattgaa accattgaat tccgaatatg 60
gtaaagtagc tcccggatgg ggaacgaccc ctttgatggg tgttgcaatg gctctatttg 120
cggtattcct atctattatt ttggagattt ataattcttc tgttctactg gatggaattt 180
cagtgaatta gactgagaag aatcttgaag ttctagcttt tagctcgata caaaaaagta 240
aagtatgcag gtctaacaat tttagcctat tctcctttgg tagttcgacc gcgaaatttt 300
tttctgcatt gtatatttcc gga 323